(19) Europäisches Patentamt
European Patent Office
Office européen des brevets

(11) Publication number: **0 280 841**
**A2**

(12) # EUROPEAN PATENT APPLICATION

(21) Application number: **88100079.8**

(22) Date of filing: **05.01.88**

(51) Int. Cl.[4]: **C07F 7/18**, C07F 7/08, A61K 31/695

(30) Priority: **05.01.87 JP 17/87**

(43) Date of publication of application: **07.09.88 Bulletin 88/36**

(84) Designated Contracting States: **CH DE FR GB IT LI**

(71) Applicant: **SANWA KAGAKU KENKYUSHO CO., LTD.**
**No. 35, Higashi-sotobori-cho**
**Higashi-ku Nagoya-shi Aichi-ken(JP)**

(72) Inventor: **Kurono, Masayasu Sanwa Kagaku Kenkyusho Co., Ltd.**
**35, Higashi-sotobori-cho**
**Higashi-ku, Nagoya Aichi-ken(JP)**
Inventor: **Unno, Ryoichi Sanwa Kagaku Kenkyusho Co., Ltd.**
**35, Higashi-sotobori-cho**
**Higashi-ku, Nagoya Aichi-ken(JP)**
Inventor: **Kimura, Hiromoto Sanwa Kagaku Kenkyusho Co., Ltd.**
**35, Higashi-sotobori-cho**
**Higashi-ku, Nagoya Aichi-ken(JP)**
Inventor: **Ozawa, Hiroshi Sanwa Kagaku Kenkyusho Co., Ltd.**
**35, Higashi-sotobori-cho**
**Higashi-ku, Nagoya Aichi-ken(JP)**
Inventor: **Mitani, Takahiko Sanwa Kagaku Kenkyusho Co., Ltd.**
**35, Higashi-sotobori-cho**
**Higashi-ku, Nagoya Aichi-ken(JP)**
Inventor: **Jomori, Takahito Sanwa Kagaku Kenkyusho Co., Ltd.**
**35, Higashi-sotobori-cho**
**Higashi-ku, Nagoya Aichi-ken(JP)**
Inventor: **Koketsu, Masahiko Sanwa Kagaku Kenkyusho Co., Ltd.**
**35, Higashi-sotobori-cho**
**Higashi-ku, Nagoya Aichi-ken(JP)**
Inventor: **Michishita, Hisashi Sanwa Kagaku Kenkyusho Co. Ltd**
**35, Higashi-sotobori-cho**
**Higashi-ku, Nagoya Aichi-ken(JP)**
Inventor: **Sawai, Kiichi Sanwa Kagaku Kenkyusho CO. Ltd.**
**35, Higashi-sotobori-cho**
**Higashi-ku, Nagoya Aichi-ken(JP)**

**EP 0 280 841 A2**

(74) Representative: **Wächtershäuser, Günter, Dr.**
**Tal 29**
**D-8000 München 2(DE)**

(54) **Organo-silicone compounds and use thereof.**

(57) An organo-silicone compound of the formula

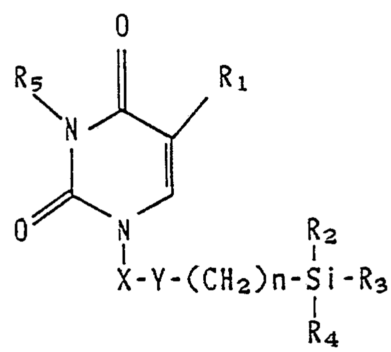

wherein X is a methylene or carbonyl radical, Y is an NH or O, n is an integer, $R_1$ is a hydrogen atom, halogen atom or alkyl group, $R_2$, $R_3$ and $R_4$ are an alkyl group, alkoxy group, phenyl radical or substituted phenyl radical, respectively, and $R_5$ a is hydrogen atom or a group of

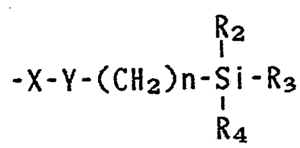

in which X, Y, n, $R_2$, $R_3$ and $R_4$ have the meanings as referred to, and a use of the compound for an anti-tumor composition.

## Organo-silicone compounds and use thereof

The present invention relates to novel organo-silicone compounds, a process for the preparation thereof, and an anti-tumor composition containing the compound as an effective ingredient therefor.

As organo-silicone compounds having an anti-tumor activity, silatrane compounds have been known by a toxicity thereof prohibits the use of such compounds for the therapeutical purpose [ "Top Curr. Chem." Vol.84, page 77 - 135 (1979)].

While, 5-fluorouracil (5-FU), Tegafur and Carmofur may be listed as exemplar organo-silicone compounds which have been actually or clinically employed for curing tumors.

Among the clinically employed compounds the 5-FU has an excellent anti-tumor activity but an oral dosage thereof shows a high toxicity in that it often causes a certain trouble in digestive canals, and thus this compond has exclusively been administrated by injection. While, the Tegafur shows a lower toxicity in oral dosage, but has a disadvantage in that anti-tumor activity thereof is also low. The Carmofur has a disadvantage in that side effects will appear in central nervous system to give a thermal feeling and increase a pollakiurea.

The present inventors have also studied and investigated various organo-silicone compounds, in view of an anti-tumor activities thereof to report amine derivatives containing a silicon atom in molecule thereof (USP 4560679 and Jap. Unexamined Pat. Appln. Gazette No. 155390/1986), and carbamoyl derivatives containing a silicon atom in molecule thereof (Jap. Unexamined Pat. Appln. Gazette Nos. 98091/1984, 65890/1986 and 10094/1987).

These organo-silicone compounds reported by the inventors are really effective as an anti-tumor agent and show a relatively low toxicity, but it has been highly required to develop more excellent one, in various view points.

In view of the above, the inventors have continued their study and investigation to discover more effective compounds having a higher anti-tumor activity and showing no or lower toxicity, and which characteristics will not be affected by a form for administration, in order to dissolve such a conflicting problem between a level of anti-tumor activity and a safety in actual use, which has been encountered in the conventional anti-tumor agents.

One of the objects of the invention, therefore, lies in providing such compounds.

Another object of the invention is to provide a process for the preparation of such compounds.

Other object of the invention is to provide an anti-tumor composition containing as an effective ingredient, at least one of such compounds.

According to the invention, the first or main object of the invention is attained by an organo-silicone compound of the formula

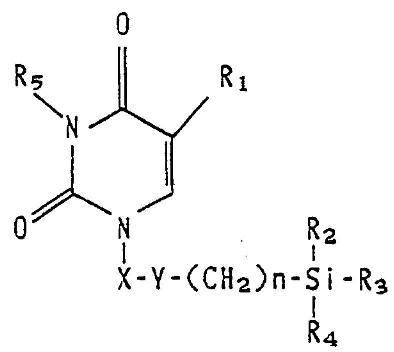

(I)

wherein X is a methylene or carbonyl radical, Y is an NH or O, n is an integer, $R_1$ is a hydrogen atom, halogen atom or alkyl group, $R_2$, $R_3$ and $R_4$ are an alkyl group, alkoxy group, phenyl radical or substituted phenyl radical, respectively, and $R_5$ is a hydrogen atom or a group of

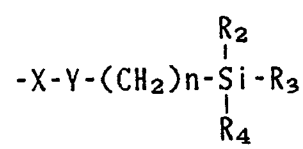

in which X, Y, n, $R_2$, $R_3$ and $R_4$ have the meanings as referred to.

In connection with the compounds (I), the definition of each substituent shall be given as follows. The halogen may be of fluorine, chlorine, bromine or iodine but fluorine is preferable. The alkyl group may be of straight-chain alkyl radicals, branched-chain alkyl radicals or cycloalkyl radicals. As the straight-chain alkyl radicals, one having 1 to 10 carbon atoms, for instance methyl, ethyl, n-propyl, n-butyl, n-pentyl, n-hexyl, n-heptyl, n-decyl and the like may be listed. As the branched-chain alkyl radicals, isopropyl, isobutyl, s-butyl, t-butyl, isopentyl and the like may be listed. As the cycloalkyl radicals, cyclopropyl, cyclobutyl, cyclopentyl, cyclohexyl and the like may be listed. As the alkoxy group, methoxy, ethoxy, 2-methoxyethoxy and the like may be listed. As the substituent for phenyl radical, p-chloro, p-bromo, p-methyl, p-methoxy or the like may be listed.

According to the process for the invention, the compounds shown by said Formula (I) can be prepared by reacting a compound of the formula

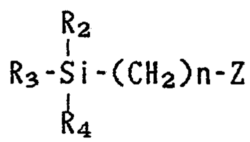

(II)

wherein $R_2$, $R_3$, $R_4$ and n have the meanings as referred to, and Z is a radical of $-NH_2$, $-N=C=O$ or $-OCH_2Cl$,
with a compound of the formula

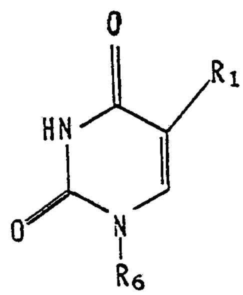

(III)

wherein $R_1$ has the meaning as referred to, and $R_6$ is a hydrogen atom, alkali metal or a radical of -COCl.

Referring to the process in more detail, there are following three synthetic routes.

Route A

According to this route, a silylalkylamine of the formula

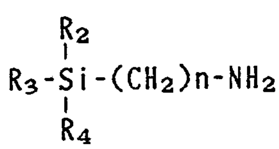

(II-a)

wherein $R_2$, $R_3$, $R_4$ and n have the meanings as referred to,
is reacted with a 1-chloroformyl-5-substituted uracil of the formula

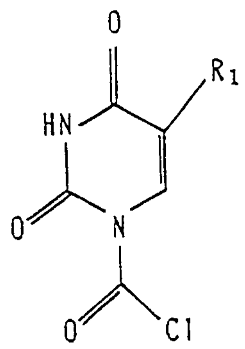

(III-a)

wherein $R_1$ has the meaning as referred to.

This reaction proceeds only by stirring the both raw materials in equi-molar amount, in the presence of a solvent. As the solvent, pyridine, N,N-dimethylformamide, dimethylsulfoxide, hexamethylphosphoryl-triamide or the like may be employed. The reaction temperature depends upon the raw materials and kind of the solvent, but a range of 0 to 10°C is preferable, since the 1-chloroformyl-5-substituted uracil as one of the raw materials is, in general, not so stable. The 1-chloroformyl-5-substituted uracil can be prepared in accordance with the method as disclosed in "Bull. Chem. Soc. Japan" Vol. 50, pages 2406 -2412 (1977). Trimethylsilylalkylamines can be prepared from trimethylsilylalkylalcohols, which are synthetheisized in accordance with the method as disclosed in "J. Amer. Chem. Soc." Vol. 74, page 1003 (1952), by a conversion into trimethylsilylalkylhalides with a suitable halogenating reagent, followed by the Gabriel synthesis or a reduction of nitrides derived from the halides.

Route B

According to this route, an isocyanate of the formula

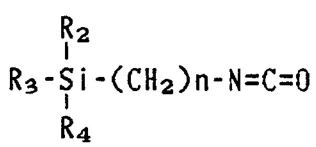

(II-b)

wherein $R_2$, $R_3$, $R_4$ and n have the meanings as referred to, is reacted with a 5-substituted uracil of the formula

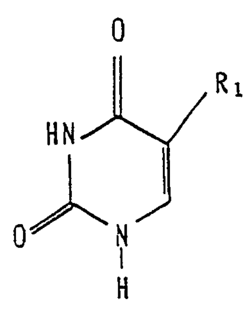

(III-b)

wherein $R_1$ has the meaning as referred to.

This reaction proceeds also by only stirring the both raw materials in equi-molar amount, in the presence of a solvent. As the solvent, those as described on Route A can be employed. The reaction temperature depends on the raw materials and kind of the solvent, but a range of 0 to 100°C is generally selected.

The isocyanate shown by the formula II-b can be prepared by reacting the compound of the formula II-a with phosgene in molar amount of 1.0 to 3.0 times.

Route C

According to this route, a compound of the formula

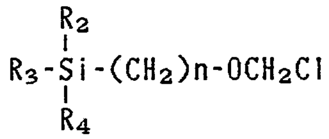

(II-c)

wherein $R_2$, $R_3$, $R_4$ and n have the meanings as referred to, is reacted with a compound of the formula

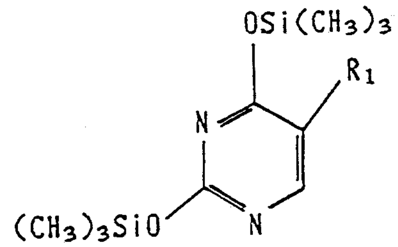

(IV)

wherein $R_1$ has the meaning as referred to.

The reaction will proceed only by stirring the both raw materials, in the presence of a solvent, when the compound II-c is employed in molar amount of 1 to 2 times to the other raw material, but it can be accelerated by adding sodium iodide or a Lewis acid (boron trifluoride, titanium tetrachloride, tin tetrachloride or the like) in catalytic amount. As the solvent, acetonitrile, dichloromethane, chloroform or the like can be employed. The reaction temperature depends upon the raw materials and kind of the solvent but a range of 0 to 40°C is preferably selected.

The α-chloroether shown by the formula II-c can be prepared in accordance with a known method as disclosed in "Chem. Rev." Vol. 55, page 301 (1955). While, the compound shown by the formula IV has been known or can be prepared in accordance with the method as disclosed in BP 1168391.

In case of preparing an anti-tumor composition with use of at least one of the organo-silicone compounds, as effective ingredient(s), there is no limitation in its medicine form and thus it may be made into one for oral or none-oral administration. As for oral administration, tablet, capsule, granule, powder and the like may be listed. While, as for none-oral administration, a suppository may exemplarly be listed. In connection with this, the medicine can be prepared in conventional manner. An amount for administrating the compound for human depends on kind of the compound selected, condition of illness, age of a patient, form of the medicine and other factors but in general case for an adult, 300 to 5000mg/day in oral administration, or 100 to 2000mg/day in suppository is preferable.

The invention will now be further explained with reference to Examples for preparing the compounds according to the invention, Pharmacological Test Example as well as Examples for preparing medicines.

Example 1

1-[3-(trimethylsilyl)propylcarbamoyl]-5-fluorouracil (Compound 1)

To 13.0g (0.100mol) of 5-fluorouracil in 400ml of anhydrous pyridine was added dropwise 9.90g (50.0mmol) of trimethylchloroformate with cooling on ice-bath at 5°C. The mixture was stirred for 1 hour at 5°C and then unreacted phosgene was removed in vacuo. To the reaction mixture was added dropwise 6.57g (50.0mmol) of 3-(trimethylsilyl)propylamine and the mixture was stirred for 1 hour at 5 to 10 °C and then the solvent was evaporated in vacuo. The resulting residue was dissolved in 300ml of chloroform and 300ml of 3% hydrochloric acid and the chloroform layer was washed with water, dried over sodium sulfate and concentrated in in vacuo. The resulting crude crystals was recrystallized with ether to afford 13.0g of the desired compound (Yield : 90.4%).

Melting point : 146 - 149°C

Elementary analysis : $C_{11}H_{18}FN_3O_3Si$

Cal. : H 6.31; C 45.98; N 14.62

Found : H 6.41; C 46.14; N 14.77
IR spectrum ($\nu_{max}^{KBr}$) $cm^{-1}$ :
3300, 3200, 3100 ($\nu_{NH}$), 3050, 2960, 2830 ($\nu_{CH}$), 1765, 1730, 1690 ($\nu_{C=O}$), 1535 ($\delta_{NH}$), 1250 ($\nu_{C-Si}$)
NMR spectrum ($CDCl_3$) $\delta$ ppm :
- 0.01 (9H, s, $-SiMe_3$)
0.3 - 0.7 (2H, m, $-C\underline{H_2}-Si-Me_3$)
1.3 - 1.9 (2H, m, $-C\underline{H_2}CH_2SiMe_3$)
3.2 - 3.6 (2H, m, - $NHC\underline{H_2}-$)
8.59 (1H, s, J = 7.0Hz, $C_6$-H)
8.9 - 9.4 (1H, br., -CONH-)
MS spectrum (EI/DI) m/z :
272 ($M^+$-Me), 100 (base peak)

Example 2

1-[4-(trimethylsilyl)butylcarbamoyl]-5-fluorouracil (Compound 2)

The procedure described in Example 1 was repeated except that 7.25g (50.0mmol) of 4-(trimethylsilyl)-butylamine was employed in place of 3-(trimethylsilyl)propylamine, to obtain 13.4g of the desired compound (Yield : 89.2%).

Melting point : 129 - 132°C
Elementary analysis : $C_{12}H_{20}FN_3O_3Si$
Cal. : H 6.69; C 47.82; N 13.94
Found : H 6.83; C 47.62; N 13.91
IR spectrum ($\nu_{max}^{KBr}$) $cm^{-1}$ :
3330, 3200, 3090 ($\nu_{NH}$), 2970, 2950 ($\nu_{CH}$), 1740, 1670 ($\nu_{C=O}$), 1510 ($\delta_{NH}$), 1250 ($\nu_{C-Si}$)
NMR spectrum ($CDCl_3$) $\delta$ ppm :
- 0.03 (9H, s, $-SiMe_3$)
0.3 - 0.7 (2H, m, $-C\underline{H_2}SiMe_3$)
1.1 - 1.9 (4H, m, $-(\underline{CH_2})_3CH_2SiMe_3$)
3.2 - 3.6 (2H, m, $-NHC\underline{H_2}-$)
8.59 (1H, d, J = 7.0Hz, $C_6$-H)
8.9 - 9.3 (1H, br., -CONH-)
MS spectrum (EI/DI) m/z :
286 ($M^+$-Me), 100 (base peak)

Example 3

1-[5-(trimethylsilyl)pentylcarbamoyl]-5-fluorouracil (Compound 3)

The procedure described in Example 1 was repeated except that 7.95g (50.0mmol) of 5-(trimethylsilyl)-pentylamine was employed in place of 3-(trimethylsilyl)propylamine, to obtain 13.7g of the desired compound (Yield : 91.1%).

Melting point : 118 - 119°C
Elementary analysis : $C_{13}H_{22}FN_3O_3Si$
Cal. : H 7.03; C 49.50; N 13.32
Found : H 7.22; C 49.52; N 13.32
IR spectrum ($\nu_{max}^{KBr}$) $cm^{-1}$ :
3336, 3188 ($\nu_{NH}$), 1738, 1680 ($\nu_{C=O}$), 1503 ($\delta_{NH}$), 1250 ($\nu_{C-Si}$)
NMR spectrum ($CDCl_3$) $\delta$ ppm :
- 0.12 (9H, s, $-SiMe_3$)
0.1 - 0.6 (2H, m, $-C\underline{H_2}SiMe_3$)
0.9 - 1.7 (6H, m, $-(\underline{CH_2})_3CH_2SiMe_3$)

3.1 - 3.5 (2H, m, $-NHCH_2-$)
7.5 - 7.9 (1H, m, -CONH-)
8.48 (1H, d, J = 7.0Hz, $C_6$-H)
8.8 - 9.2 (1H, m, $N_3$-H)

Example 4

1-[7-(trimethylsilyl)heptylcarbamoyl]-5-fluorouracil (Compound 4)

The procedure described in Example 1 was repeated except that 9.35g (50.0mmol) of 7-(trimethylsilyl)-heptylamine was employed in place of 3-(trimethylsilyl)propylamine, to obtain 15.5g of the desired compound (Yield : 90.5%).

Melting point : 118 - 119°C
Elementary analysis : $C_{15}H_{26}FN_3O_3Si$
Cal. : H 7.63; C 52.45; N 12.23
Found : H 7.90; C 52.60; N 12.36
IR spectrum ($\nu^{KBr}_{max}$) $cm^{-1}$ :
3334, 3186 ($\nu_{NH}$), 1737, 1680 ($\nu_{C=O}$), 1503 ($\delta_{NH}$), 1250 ($\nu_{C-Si}$)
NMR spectrum ($CDCl_3$) $\delta$ ppm :
- 0.17 (9H, s, $-SiMe_3$)
0.1 - 0.6 (2H, m, $-CH_2SiMe_3$)
1.1 - 1.5 (10H, m, $-(CH_2)_5CH_2SiMe_3$
3.1 - 3.5 (2H, m, $-NHCH_2-$)
8.48 (1H, d, J = 7.0Hz, $C_6$-H)
8.9 - 9.1 (1H, m, -CONH-)

Example 5

1-[2-(trimethylsilyl)ethyloxymethyl]-5-fluorouracil (Compound 5a) and 1,3-bis[2-(trimethylsilyl)-ethyloxymethyl]-5-fluorouracil (Compound 5b)

To a mixture of 2.74g (10.0mmol) of 2.4-bis(trimethylsilyl)-5-fluorouracil and 750mg (5.00mmol) of sodium iodide in 35.0ml of anhydrous acetonitrile was added dropwise 2.50g (15.0mmol) of 2-(trimethylsilyl)ethyloxymethyl chloride to maintain a temperature of the solution at 20°C. After stirring the mixturefor 10 minutes at 20°C, the solvent was evaporated in vacuo, and the resulting residue was dissolved in 100ml of chloroform and 100ml of water and the chloroform layer was dried over sodium sulfate and concentrated in vacuo. The resulting residue was crustallized with a small amount of n-hexane to afford the 1.12g (43.1%) of the desired compound (5a).

While, the filtrate was concentrated and the resulting residue was purified by silica-gel chromatography (developing solvent : chloroform/methanol = 300/1) to obtain further 0.39g (15,0%) of the compound 5a as well as 1.44g (36.9%) of the other desired compound (5b).

Compound 5a

Melting point : 132 - 133°C
Elementary analysis : $C_{10}H_{17}FN_2O_3Si$
Cal. : H 6.58; C 46.14; N 10.76
Found : H 6.74; C 46.42; N 10.87
IR spectrum ($\nu^{KBr}_{max}$) $cm^{-1}$ :
1715, 1690, 1660 ($\nu_{C=O}$), 1250 ($\nu_{C-Si}$), 1090 ($\nu_{C-O}$)
NMR spectrum ($CDCl_3$) $\delta$ ppm :
- 0.11 (9H, s, $-SiMe_3$)
0.85 (2H, t, J = 8.0Hz, $-CH_2SiMe_3$)
0.58 (2H, t, J = 8.0Hz, $-OCH_2CH_2-$)
5.10 (2H, s, $-OCH_2N<$ )

7.39 (1H, d, J = 5.0Hz, $C_6$-H)
9.83 (1H, br., -NH-)
MS spectrum (EI/DI) m/z :
260 ($M^+$), 187 (base peak)

Compound 5b

IR spectrum ($\nu^{KBr}_{max}$) $cm^{-1}$ :
1725, 1690, 1670 ($\nu_{C=O}$), 1250 ($\nu_{C-Si}$), 1090 ($\nu_{C-O}$)
NMR spectrum ($CDCl_3$) $\delta$ ppm :
- 0.10 (18H, s, $-SiMe_3$ x 2)
0.84 (4H, t, J = 8.0Hz, $-C\underline{H_2}SiMe_3$ x 2)
3.4 - 3.8 (4H, m, $-OC\underline{H_2}CH_2$-x 2)
5.10 (2H, s, $-OCH_2N$ < )
5.37 (2H, s, $-OCH_2N$ < )
7.36 (1H, d, J = 5.0Hz, $C_6$-H)
MS spectrum (EI/DI) m/z :
375 (M-Me), 73 (base peak)
High MS spectrum (m/z) :
375.1584 (M-Me, $C_{15}H_{28}FN_2O_4Si_2$; Cal. 375.1571)

Example 6

1-[3-(trimethylsilyl)propyloxymethyl]-5-fluorouracil (Compound 6a) and 1,3-bis[3-(trimethylsilyl)-propyloxymethyl]-5-fluorouracil (Compound 6b)

The procedure described in Example 5 was repeated except that 2.73g (15.0mmol) of 3-(trimethylsilyl)-propyloxymethyl chloride was employed in place of 2-(trimethylsilyl)ethyloxymethyl chloride, to obtain 1.70g (61.7%) and 0.82g (19.5%) of the desired compounds 6a and 6b, respectively.

Compound 6a

Melting point : 119 - 120°C
Elementary analysis : $C_{11}H_{19}FN_2O_3Si$ Cal. : H 6.98; C 48.15; N 10.21
Found : H 7.24; C 47.98; N 10.16
IR spectrum ($\nu^{KBr}_{max}$) $cm^{-1}$ :
1700, 1660 ($\nu_{C=O}$), 1250 ($\nu_{C-Si}$
NMR spectrum ($CDCl_3$ $\delta$ ppm :
- 0.17 (9H, s, $-SiMe_3$)
0.2 - 0.5 (2H, m, $-C\underline{H_2}SiMe_3$)
1.2 - 1.8 (2H, m, $-C\underline{H_2}CH_2SiMe_3$)
3.39 (2H, t, J = 7.0Hz, $-CH_2C\underline{H_2}O$-)
5.04 (2H, s, $-OCH_2N$ < )
7.34 (1H, d, J = 5.0Hz, $C_6$-H)
9.75 (1H, br., -NH-)
MS spectrum (EI/DI) m/z :
260 (M-Me), 73 (base peak)

Compound 6b

IR spectrum ($\nu^{KBr}_{max}$) $cm^{-1}$ :
1725, 1690, 1670 ($\nu_{C=O}$), 1250 ($\nu_{C-Si}$)
NMR spectrum ($CDCl_3$) $\delta$ ppm :
- 0.10 (18H, s, $-SiMe_3$ x 2)

0.2 - 0.6 (4H, m, $-\underline{CH_2}SiMe_3 \times 2$) 1.2 - 1.8 (4H, m, $-\underline{CH_2}CH_2SiMe_3 \times 2$)
3.3 - 3.7 (4H, m, $-CH_2\underline{CH_2}- \times 2$)
5.13 (2H, s, $-OCH_2N<$)
5.41 (2H, s, $-OCH_2N<$)
7.36 (1H, d, J = 5.0Hz, $C_6$-H)
MS spectrum (EI/DI) m/z :
418 ($M^+$), 403 (M-Me) 73 (base peak)
High MS spectrum (m/z) :
418.2142 ($M^+$, $C_{18}H_{35}FN_2O_4Si_2$; Cal. 418.2120)

Example 7

1-[5-(trimethylsilyl)pentyloxymethyl]-5-fluorouracil (Compound 7a) and 1,3-bis[5-(trimethylsilyl)-pentyloxymethyl]-5-fluorouracil (Compound 7b)

The procedure described in Example 5 was repeated except that 3.13g (15.0mmol) of 5-(trimethylsilyl)-pentyloxymethyl chloride was employed in place of 2-(trimethylsilyl)ethyloxymethyl chloride, to obtain 2.22g (73.4%) and 1.10g (23.2%) of the desired compounds 7a and 7b respectively.

Compound 7a

Melting point : 86 - 87°C
Elementary analysis : $C_{13}H_{23}FN_2O_3Si$
Cal. : H 7.67; C 51.63; N 9.26
Found : H 7.78; C 51.33; N 9.25
IR spectrum ($\nu_{max}^{KBr}$) $cm^{-1}$ :
1700, 1665 ($\nu_{C=O}$), 1250 ($\nu_{C\text{-}Si}$)
NMR spectrum ($CDCl_3$) $\delta$ ppm :
- 0.14 (9H, s, $-SiMe_3$)
0.2 - 0.6 (2H, m, $-\underline{CH_2}SiMe_3$)
1.0 - 1.7 (6H, m, $-(\underline{CH_{23}}CH_2SiMe_3)$)
3.3 - 3.6 (2H, m, $-O\underline{CH_2}CH_2-$)
5.11 (2H, s, $-OCH_2N<$)
7.39 (1H, d, J = 5.0Hz, $C_6$-H)
9.83 (1H, br., -NH-)
MS spectrum (EI/DI) m/z :
287 (M-Me), 73 (base peak)

Compound 7b

IR spectrum ($\nu_{max}^{KBr}$) $cm^{-1}$ :
1730, 1670 ($\nu_{C=O}$), 1250 ($\nu_{C\text{-}Si}$)
NMR spectrum ($CDCl_3$) $\delta$ ppm :
- 0.09 (18H, s, $-SiMe_3 \times 2$)
0.2 - 0.6 (4H, m, $-\underline{CH_2}SiMe_3 \times 2$)
1.0 - 1.7 (12H, m, $-\underline{(CH_2)_3}CH_2SiMe_3 \times 2$)
3.3 - 3.8 (4H, m, $-O\underline{CH_2}CH_2- \times 2$)
5.12 (2H, s, $-OCH_2N<$)
5.39 (2H, s, $-OCH_2N<$)
7,35 (1H, d, J = 5.0Hz, $C_6$-H)
MS spectrum (CI/DI) m/z :
475 ($M^+$ + 1, base peak)
High MS spectrum (m/z) :
474.2763 ($M^+$, $C_{22}H_{43}FN_2O_4Si_2$; Cal. 474.2746)

Pharmacological Test Example 1

(Anti-tumor activity to Lewis lung carcinoma

Under a skin of female $BDF_1$ strain mice (age of 6 weeks), Lewis lung carcinoma cells ($2.5 \times 10^5$) were transplanted to form of a tumor. Each of testing compoundswas orally administrated to the animals 3 times (at 4th, 8th and 10th day after the transplantation, respectively) and then each of the animals was killed at 17th day after the transplantation to measure a weight of the tumor.

An anti-tumor activity of each testing compound was calculated based on following equation, as an inhibition of tumor proliferation.

$$\text{Inhibition of proliferation (\%)} = \frac{C - T}{C} \times 100$$

wherein T : Weight of tumor in the testing compound administrated group, and
C : Weight of tumor in the control group giving no testing compound.

Results are shown in following Table.

TABLE

| Compound | Dose/day x 3 (mg/kg) | Inhibition of proliferation (%) |
|---|---|---|
| 1 | 239<br>398<br>557 | 86<br>100<br>100 |
| 2 | 250<br>417<br>584 | 82<br>99<br>100 |
| 3 | 282<br>437<br>612 | 45<br>88<br>97 |
| 4 | 285<br>476<br>666 | 59<br>97<br>100 |

Phamacological Test Example 2

(Acute toxicity)

Female ICR strain mice (age of 5 weeks) were used for this experiment. The compound 2 suspended in 1% Tween 80 - 0.5% CMC solution was given to each animal orally. General condition, behaviour, body weight and mortality were observed daily throughout the experimental period of 14 days.

Then, a value of $LD_{50}$ was calculated in accordance with the Litchfield-Wilcoxon method to find as 820mg/kg.

Medicine Preparation Example 1 (Tablet)

Following ingredients were prescripted and treated in a conventional manner to prepare tablets.

| | |
|---|---|
| Compound 1 | 100 (mg) |
| Crystalline cellulose | 20 |
| Lactose | 41 |
| Corn starch | 30 |
| Hydroxypropylcellulose | 6 |
| Magnesium stearate | 3 |
| | 200 mg/tablet |

Medicine Preparation Example 2 (Capsule)

Following ingredients were prescripted and treated in a conventional manner to prepare capsules.

| | |
|---|---|
| Compound 2 | 200 (mg) |
| Crystalline cellulose | 50 |
| Silicic anhydride | 2 |
| Magnesium stearate | 3 |
| | 255 mg/capsule |

Medicine Preparation Example 3 (Granule)

Following ingredients were prescripted and treated in a conventional manner to prepare granules.

| | |
|---|---|
| Compound 3 | 500 (mg) |
| lactose | 323 |
| Corn starch | 150 |
| Polyvinylpyrrolidone | 25 |
| Silicic anhydride | 2 |
| | 1000 mg/package |

Medicine Preparation Example 4 (Suppository)

Following ingredients were prescripted and treated in a conventional manner to prepare suppositories.

| | |
|---|---|
| Compound 4 | 300 (mg) |
| Witep-Sol W-35 (Trademark) | 1700 |
| | 2000 mg/piece |

**Claims**

1. An organo-silicone compound of the formula

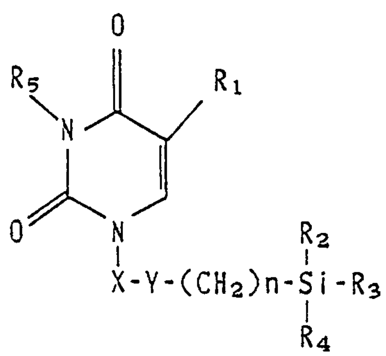

(I)

wherein X is a methylene or carbonyl radical, Y is an NH or O, n is an integer, $R_1$ is a hydrogen atom, halogen atom or alkyl group, $R_2$, $R_3$ and $R_4$ are an alkyl group, alkoxy group, phenyl radical or substituted phenyl radical, respectively, and $R_5$ is a hydrogen atom or a group of

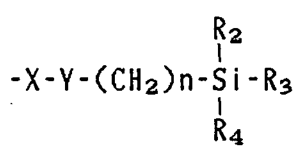

in which X, Y, n, $R_2$, $R_3$ and $R_4$ have the meanings as referred to.

2. An organo-silicone compound as claimed in Claim 1, wherein said compound is 1-[3-(trimethylsilyl)-propylcarbamoyl]-5-fluorouracil.

3. An organo-silicone compound as claimed in Claim 1, wherein said compound is 1-[4-(trimethylsilyl)-butylcarbamoyl]-5-fluorouracil.

4. An organo-silicone compound as claimed in Claim 1, wherein said compound is 1-[5-(trimethylsilyl)-pentylcarbamoyl]-5-fluorouracil.

5. An organo-silicone compound as claimed in Claim 1, wherein said compound is 1-[7-(trimethylsilyl)-heptylcarbamoyl]-5-fluorouracil.

6. An organo-silicone compound as claimed in Claim 1, wherein said compound is 1-[2-(trimethylsilyl)-ethyloxymethyl]-5-fluorouracil.

7. An organo-silicone compound as claimed in Claim 1, wherein said compound is 1-[3-(trimethylsilyl)-propyloxymethyl]-5-fluorouracil.

8. An organo-silicone compound as claimed in Claim 1, wherein said compound is 1-[5-(trimethylsilyl)-pentyloxymethyl]-5-fluorouracil.

9. An organo-silicone compound as claimed in Claim 1, wherein said compound is 1,3-bis[2-(trimethylsilyl)ethyloxymethyl]-5-fluorouracil.

10. An organo-silicone compound as claimed in Claim 1, wherein said compound is 1,3-bis[3-(trimethylsilyl)propyloxymethyl]-5-fluorouracil.

11. An organo-silicone compound as claimed in Claim 1, wherein said compound is 1,3-bis[5-(trimethylsilyl)pentyloxymethyl]-5-fluorouracil.

12. A process for the preparation of an organo-silicone compound of the formula

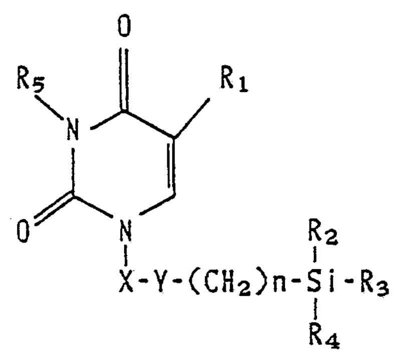

(I)

wherein X is a methylene or carbonyl radical, Y is an NH or O, n is an integer, $R_1$ is a hydrogen atom, halogen atom or alkyl group, $R_2$, $R_3$ and $R_4$ are an alkyl group, alkoxy group, phenyl radical or substituted phenyl radical, respectively, and $R_5$ is a hydrogen atom or a group of

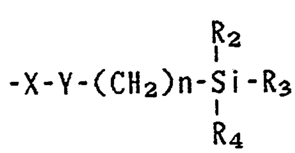

in which X, Y, n, $R_2$, $R_3$ and $R_4$ have the meanings as referred to. which comprises a step of reacting a compound of the formula

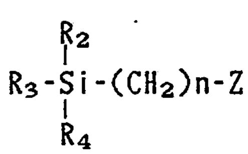

(II)

wherein $R_2$, $R_3$, $R_4$ and n have the meanings as referred to, and Z is a radical of $-NH_2$, $-N=C=O$ or $-OCH_2Cl$,
with a compound of the formula

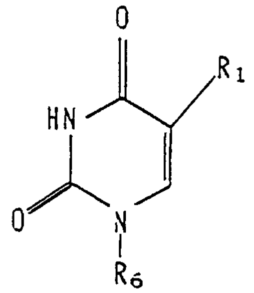

(III)

wherein $R_1$ has the meaning as referred to, and $R_6$ is a hydrogen atom, alkali metal or a radical of -COCl.

13. A process as claimed in Claim 12, wherein a silylalkylamine of the formula

$$R_3-\overset{R_2}{\underset{R_4}{Si}}-(CH_2)n-NH_2 \qquad (II\text{-}a)$$

in which $R_2$, $R_3$ and $R_4$ are an alkyl group, alkoxy group, phenyl radical or substituted phenyl radical, respectively, and n is an integer,
is reacted with a 1-chloroformyl-5-substituted uracil of the formula

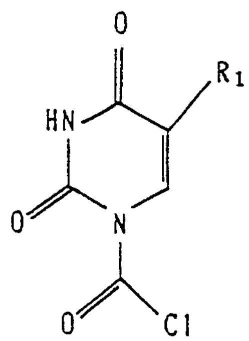

(III-a)

in which $R_1$ is a hydrogen atom, halogen atom or alkyl group.

14. A process as claimed in Claim 12, wherein an isocyanate of the formula

$$R_3-\overset{R_2}{\underset{R_4}{Si}}-(CH_2)n-N=C=O \qquad (II\text{-}b)$$

in which $R_2$, $R_3$, and $R_4$ are an alkyl group, alkoxy group, phenyl radical or substituted phenyl radical, respectively, and n is an integer,
is reacted with a 5-substituted uracil of the formula

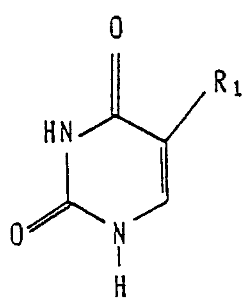

(III-b)

in which $R_1$ is a hydrogen atom, halogen atom or alkyl group.

15. A process as claimed in Claim 12, wherein a compound of the formula

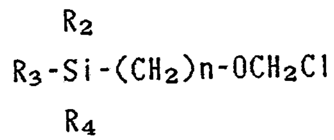

(II-c)

in which $R_2$, $R_3$ and $R_4$ are an alkyl group, alkoxy group, phenyl radical or substituted phenyl radical, respectively, and <u>n</u> is an integer,
is reacted with a compound of the formula

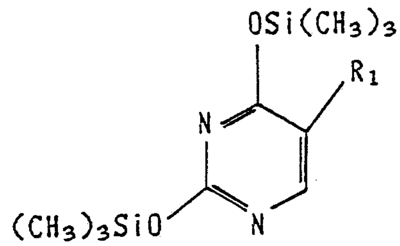

(IV)

in which $R_1$ is a hydrogen atom, halogen atom or alkyl group.

16. An anti-tumor composition comprising an effective amount of an organo-silicone compound of the formula

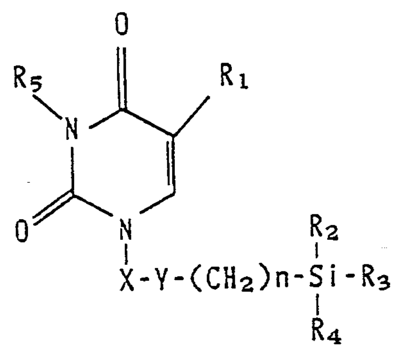

(I)

wherein X is a methylene or carbonyl radical, Y is an NH or O, <u>n</u> is an integer, $R_1$ is a hydrogen atom, halogen atom or alkyl group, $R_2$, $R_3$ and $R_4$ are an alkyl group, alkoxy group, phenyl radical or substituted phenyl radical, respectively, and $R_5$ is a hydrogen atom or a group of

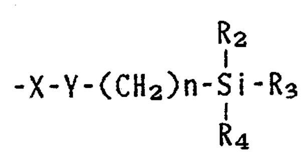

in which X, Y, <u>n</u>, $R_2$, $R_3$ and $R_4$ have the meanings as referred to, together with a pharmaceutically acceptable carrier.

17. An anti-tumor composition as claimed in Claim 16, wherein said organo-silicone compound is at least one of those selected from the group consisting of

a) 1-[3-(trimethylsilyl)propylcarbamoyl]-5-fluorouracil,
b) 1-[4-(trimethylsilyl)butylcarbamoyl]-5-fluorouracil,
c) 1-[5-(trimethylsilyl)pentylcarbamoyl]-5-fluorouracil,
d) 1-[7-(trimethylsilyl)heptylcarbamoyl]-5-fluorouracil,
e) 1-[2-(trimethylsilyl)ethyloxymethyl]-5-fluorouracil,

f) 1-[3-(trimethylsilyl)propyloxymethyl]-5-fluorouracil,
g) 1-[5-(trimethylsilyl)pentyloxymethyl]-5-fluorouracil,
h) 1,3-bis[2-(trimethylsilyl)ethyloxymethyl]-5-fluorouracil,
i) 1,3-bis[3-(trimethylsilyl)propyloxymethyl]-5-fluorouracil, and
j) 1,3-bis[5-(trimethylsilyl)pentyloxymethyl]-5-fluorouracil.